# EUROPÄISCHE PATENTSCHRIFT

(11) **EP 2 506 839 B1**
(45) Veröffentlichungstag und Bekanntmachung des Hinweises auf die Patenterteilung: **16.05.2018**
(21) Anmeldenummer: 10793152.9
(22) Anmeldetag: 02.12.2010
(51) Int. Cl.: A61K 9/70, A61K 38/09, A61K 38/11

(54) **TRANSDERMALES THERAPEUTISCHES SYSTEM FÜR DIE VERABREICHUNG VON PEPTIDEN**
TRANSDERMAL THERAPEUTIC SYSTEM FOR THE ADMINISTRATION OF PEPTIDES
SYSTÈME THÉRAPEUTIQUE TRANSDERMIQUE POUR L'ADMINISTRATION DE PEPTIDES

(30) Priorität: 04.12.2009 DE 102009056746
(43) Veröffentlichungstag der Anmeldung: 10.10.2012
(73) Patentinhaber: LTS LOHMANN Therapie-Systeme AG, 56626 Andernach (DE)
(72) Erfinder: HORSTMANN, Michael, 56564 Neuwied (DE); DZEKAN, Horst, 56584 Meinborn (DE); WIEDERSBERG, Sandra, 06268 Steigra (DE)
(74) Vertreter: Schweitzer, Klaus
(86) Internationale Anmeldenummer: PCT/EP2010/007322
(87) Internationale Veröffentlichungsnummer: WO 2011/066970

(56) Entgegenhaltungen:
- WO-A2-95/30410
- WO-A2-2005/042054
- US-A1- 2002 038 101
- US-A1- 2004 033 254

## Beschreibung

Der Gegenstand der vorliegenden Erfindung ist ein Transdermales Therapeutisches System (TTS) für die Verabreichung von Peptiden und anderen hochmolekularen Molekülen. Dabei sind besonders solche Peptide geeignet, die als pharmazeutischer Wirkstoff eingesetzt werden können. Hierzu zählen in besonderer Weise die Peptidhormone.

Transdermale Therapeutische Systeme (TTS) sind seit langem als pharmazeutische Darreichungsformen bekannt. Für die transdermale Applikation von pharmazeutischen Wirkstoffen mittels TTS stellt das Stratum corneum (SC), die äußerste Schicht der Haut, in den meisten Fällen die eigentliche Barriere für die Durchlässigkeit und die Geschwindigkeit des Durchtritts des pharmazeutischen Wirkstoffs dar.

Peptide und Proteine sowie andere hochmolekulare Moleküle mit einem Molekulargewicht oberhalb von 500 Dalton - wie beispielsweise Tacrolimus, Heparin und zahlreiche Salze von Betamethason - werden aufgrund ihrer Molekülgröße und ihrer physiko-chemischen Eigenschaften im Allgemeinen transdermal nicht resorbiert.

Die meisten Peptide besitzen zudem eine geringe orale Bioverfügbarkeit und unterliegen einem starken, proteolytischem Abbau im Gastrointestinaltrakt. Daher werden Peptide gewöhnlich parenteral, unter Umgehung des Gastrointestinaltraktes, verabreicht. Hierbei handelt es sich um Injektionen oder Infusionen die unter die Haut, in den Muskel oder direkt in die Blutbahn appliziert werden.

Die transdermale Route würde hier eine nichtinvasive Alternative mit hoher Patientencompliance zu dieser invasiven, parenteralen Verabreichung bieten. Es gibt daher zahlreiche Ansätze, die Durchlässigkeit der Haut für Moleküle mit einem Molekulargewicht oberhalb von 500 Dalton zu erleichtern. Hierzu zählen in erster Linie der Einsatz von Permeationsverstärkern ("Enhancern") oder die zusätzliche Anwendung von Wärme.

Eine weitere Methode schlecht hautgängige Moleküle für die transdermale Verabreichung zugänglich zu machen besteht darin, den Durchgang eines solchen Wirkstoffs durch das Stratum corneum zu erleichtern, indem diese Schicht zuvor teilweise zerstört oder entfernt wird. Diese als "skin ablation" bezeichneten Techniken verwenden thermische oder mechanische Energie, um das Stratum corneums partiell zu zerstören oder zu entfernen und so direkte Kanäle in die lebende Epidermis zu schaffen. Die Durchlässigkeit der Haut wird erhöht und eine transdermale Resorption von hochmolekularen Molekülen kann somit ermöglicht werden.

Durch diese Vorbehandlung der Haut können zudem auch hydrophile Wirkstoffe transdermal verabreicht werden, die bisher aufgrund ihrer Hydrophilie der transdermalen Route verschlossen waren. Hier kommen beispielsweise Fentanylcitrat, Granisetron-HCl, Na-Diclofenac und Apomorphin-Sulfat in Frage. Weiterhin kann die TTS-Fläche bestehender TTS Systeme durch eine "skin ablation" Vorbehandlung der Haut bei gleichen Blutspiegeln signifikant reduziert werden.

Gewöhnlich wird durch die "skin-ablation-Technik" eine Vielzahl von Mikrokanälen durch das Stratum Corneum erzeugt, jedoch ist der prozentuale "gelöcherte" Anteil der behandelten Hautfläche relativ gering. Eine Beschreibung der "skin-ablation-Technik" mittels Laser ist in WO 2007/039646 enthalten.

Triptorelin ist ein Peptidhormon mit einem Molekulargewicht von 1311 Da. Es wird zur Behandlung von fortgeschrittenem Prostatakrebs, bei Endometriose und bei frühzeitiger Pubertät angewandt. Ein weiteres Anwendungsgebiet ist die in-vitro-Fertilisation (assistierende Fertilitätstherapie). Für diese Zwecke steht Triptorelin als fertige Injektionslösung (unter dem Markennamen Decapeptyl®, Uropeptyl Depot®), und als Trockensubstanz mit Lösungsmittel zur Herstellung einer Injektionssuspension (unter dem Markennamen Pamorelin®) zur Verfügung. Triptorelin wird dabei als Salz in Form des Diacetats bzw. des Embonats eingesetzt. Bei einer Triptorelin-Behandlung wird die gegebenenfalls kurz vor ihrer Anwendung durch den Patienten hergestellte Lösung per Injektion appliziert. Eine Triptorelin-Therapie kann über einen Zeitraum von mehreren Wochen bis Monaten anhalten und eine einmal tägliche Injektion erfordern.

Desmopressin ist ein synthetisches Analogon des Peptidhormons Vasopressin mit einem Molekulargewicht von 1.069 Da und wird als Arzneistoff (Antidiuretikum) verwendet. Desmopressin ist ein Antidiuretikum. Zusätzlich gibt es noch eine Indikation bei Enuresis nocturna (Bettnässen). Desmopressin kann auch als Antihämorrhagikum gegeben werden z. B. bei Hämophilie, urämischer Thombozytopathie oder Willebrand-Jürgens-Syndrom.
Für diese Zwecke steht Desmopressin als Tabletten, (Minirin®), Injektionslösung (Minirin parenteral®) oder als Nasenspray (Minirin®, Desmopressin TAD®) zur Verfügung. Vasopressin ist ein Peptidhormon mit einem Molekulargewicht von 1084 Da. Vasopressin wird als stark blutdrucksteigernde Substanz erfolgreich bei Patienten im Schockzustand eingesetzt. Der Diabetes insipidus centralis (ICD- 10: E23.3 und N25.1) kann durch die Gabe von Vasopressin behandelt werden.

Die bekannten Erzeugnisse besitzen jedoch einige Nachteile, die insbesondere auf die allgemein bekannte geringe Stabilität der Peptide in Lösung zurückzuführen sind.

So beträgt die Haltbarkeit fertiger Injektionslösung nur 3 Wochen. Der größte Nachteil ist allerdings die geringe Patientencompliance der Injektionsspritzen aufgrund ihrer invasiven Natur. Hochmolekulare Moleküle sind der transdermalen Applikation bislang aufgrund ihrer physikochemischen Eigenschaften verschlossen. Erst durch eine Vorbehandlung der Haut wird eine transdermale Applikation dieser Moleküle ermöglicht.

Um die Stabilität und damit die Haltbarkeit der Peptidzubereitungen zu erhöhen, wird die Injektionslösung erst direkt vor Applikation durch Mischen des trockenen Wirkstoffs (meist gefriergetrocknet) mit dem Lösungsmittel hergestellt. Der Ansatz, die Injektionssuspension erst kurz vor ihrer Anwendung durch den Patienten herstellen zu lassen beinhaltet das Risiko einer ungenauen Dosierung. Auch stellt das Abfüllen von Pulvern im großtechnischen Maßstab eine Aufgabe dar, die insbesondere bei einem so hocheffizienten Wirkstoff wie einem Peptidhormon größte Anforderungen an die Genauigkeit stellt. Daher will man in der pharmazeutischen Industrie das Arbeiten mit Festkörpern möglichst vermeiden.

Schließlich können bei der Injektion selbst Schwierigkeiten auftreten, die hauptsächlich in Schmerzen bei der Anwendung, einem Verletzungsrisiko und dem Risiko von Infektionen bestehen.

Aus der WO 95/30410 A2 ist ein TTS zur Verabreichung von Peptiden wie Vasopressin durch die Haut, welcher ein Stück Epithel entfernt wurde, bekannt. Es wird ein TTS beschrieben, das ein Trägerschicht aus Hydrogel mit dem Wirkstoff Vasopressin enthält, die einseitig mit einer metallisierten Polyesterfolie abgedeckt sind, während sie auf der anderen Seite an eine Membran aus Celluloseacetat angrenzt. Außerdem werden Siliconkleber beschrieben, die sich dazu eignen, das TTS auf der deepithalsierten Hautpartie zu befestigen. Als Hydrogele werden Hydroxyalkylcellulose oder Polyvinylalkohol genannt.

Aufgabe der vorliegenden Erfindung ist es, ein Transdermales Therapeutisches System (TTS) für die Verabreichung von Peptiden und anderen schlecht hautgängigen Molekülen zur Verfügung zu stellen.

Damit das TTS bei Raumtemperatur lagerstabil ist und wenig mikrobiell anfällig, sollte es möglichst wenig Wasser enthalten.

Dabei soll das TTS auf ein Hautareal aufgebracht werden, von dem zuvor mindest ein Teilbereich des Stratum corneums zerstört oder entfernt wurde.

Es soll insbesondere ein TTS mit einem der Wirkstoffe Triptorelin, Desmopressin, Vasopressin oder eines ihrer pharmazeutisch akzeptablen Salze hergestellt werden, mit dem Peptide durch die Haut in therapeutischen Dosen an einen Patienten verabreicht werden kann.

Bei der Haut soll es sich vorzugsweise um "ablativ" vorbehandelte Haut handeln, bei der ein Anteil des Stratum Corneum entfernt worden ist.

Dabei soll nicht nur der Weg der Verabreichung mittels Injektion vermieden werden. Auch das TTS selbst soll möglichst ohne Mikroinjektionsnadeln, Mikromesser und / oder sonstige Nadeln und Widerhaken ausgestattet sein, um eine zusätzliche mechanische Verletzung des Stratum corneums zu vermeiden bzw. auszuschließen. Es kann aber gegebenenfalls mit derartigen Konstruktionselementen ausgerüstet sein.

Auch soll das Peptid im Rahmen einer Langzeitanwendung mit Hilfe des Transdermalen Therapeutischen Systems appliziert werden können.

Auch soll das Erzeugnis auf einfache und kostengünstige Weise herstellbar sein, ohne dass die für pulverförmige Darreichungsformen typischen Herstellungsprobleme berücksichtigt werden müssen.

Gelöst wird die Aufgabe durch ein Transdermales Therapeutisches System (TTS) zur Verabreichung eines Peptids auf ablativ behandelte Haut an einen Patienten, umfassend eine Rückschicht, eine Schutzfolie und mindestens eine wirkstoffhaltige Schicht, die mindestens ein Peptid und eine hydrophile, wasserlösliche Trägersubstanz enthält, die ausgewählt ist aus der Gruppe bestehend aus Polyvinylpyrrolidon, insbesondere ein Polyvinylpyrrolidon mit einem Molekulargewicht zwischen 790.000 bis 1.500.000 Da, quervernetztes Polyvinylpyrrolidon, Polyvinylalkohol-Polyvinylpyrrolidon-Mischpolymerisaten, Polysacchariden und Mischungen davon, wobei die wirkstoffhaltige Schicht einen Wassergehalt von unterhalb 20 % aufweist.

Das TTS kann weiterhin eine Rückschicht enthalten, die für das Peptid undurchlässig ist.

Die wirkstoffhaltige Schicht, die das Peptid und die Trägersubstanz für das Peptid enthält, kann haftklebend ausgerüstet sein.

Die wirkstoffhaltige Schicht lässt sich vollflächig, durch für TTS gängige Beschichtungs- und Trocknungsprozesse, herstellen.

Die wirkstoffhaltige Schicht kann als hydrophile Trägersubstanz Polyvinylpyrrolidon (PVP) enthalten. Besonders geeignet ist PVP, wie beispielsweise Kollidon 90.

Der mindestens einen wirkstoffhaltigen Schicht können weichmachende Stoffe, wie Glycerin, mittelkettige Triglyceride (Mygliol®) oder andere Kohlenwasserstoffe zugesetzt werden.

Die wirkstoffhaltige Schicht kann weitere, den Wirkstoff stabilisierende Hilfsstoffe enthalten, vorzugsweise Puffersubstanzen oder Zucker.

Das TTS kann auch mindestens eine weitere zusätzliche Haftkleberschicht enthalten, die im Wesentlichen frei von Wirkstoff und haftklebend ist. Eine solche zusätzliche Haftkleberschicht sorgt in dem Fall, dass die wirkstoffhaltige Schicht nicht oder nicht ausreichend genug haftklebend ist, für eine sichere Haftung des TTS auf der Haut.

Das TTS kann peptidische Wirkstoffe enthalten, insbesondere Peptide mit einem niedrigen Molekulargewicht kleiner als 2.500 Da.

In einer besonderen Ausführungsform enthält das TTS den Wirkstoff Triptorelin und/oder mindestens eines seiner pharmazeutisch akzeptablen Salze.

In einer weiteren besonderen Ausführungsform enthält das TTS den Wirkstoff Desmopressin und/oder mindestens eines seiner pharmazeutisch akzeptablen Salze.

In einer weiteren besonderen Ausführungsform enthält das TTS den Wirkstoff Vasopressin und/oder mindestens eines seiner pharmazeutisch akzeptablen Salze.

Ein "Transdermales Therapeutisches System" (TTS) ist ein schichtförmig aufgebautes Erzeugnis. In seiner einfachsten Ausführungsform besteht es aus einer Rückschicht, einer wirkstoffhaltigen Schicht und einer Schutzfolie, die die wirkstoffhaltige Schicht bis zur Anwendung des TTS abdeckt. Bei einer solch einfachen Konstruktion ist die wirkstoffhaltige Schicht vorzugsweise haftklebend ausgerüstet. Wenn die Klebkraft der wirkstoffhaltigen Schicht jedoch ungenügend ist, kann das TTS eine zusätzliche Haftkleberschicht aufweisen.

Diese zusätzliche Haftkleberschicht kann zwischen der wirkstoffhaltigen Schicht und der
Schutzfolie angeordnet sein.

In einer bevorzugten Ausführungsform ist die zusätzliche Haftkleberschicht zwischen der wirkstoffhaltigen Schicht und der Rückschicht angebracht. In diesem Fall überragt die Haftkleberschicht die wirkstoffhaltige Schicht an zumindest einem Abschnitt entlang des/der seitlichen Rands/Ränder der wirkstoffhaltigen Schicht. Die zusätzliche Haftkleberschicht sorgt dann während der Anwendung des TTS für eine sichere Haftung auf der Haut.

Das TTS kann auch eine Membran besitzen, die die Geschwindigkeit des Austritts des Wirkstoffs aus der wirkstoffhaltigen Schicht kontrolliert. Die Membran ist daher auf der Seite der wirkstoffhaltigen Schicht angebracht, die während der Anwendung des TTS der Haut zugewandt ist.

Das TTS selbst kann schließlich eine Nadelschicht besitzen, die direkt mit der Haut in Kontakt kommt und an seiner Unterseite mit Mikroinjektionsnadeln (= Hohlnadeln für den Durchfluss von Wirkstoff), Mikromessern (zum Anritzen der obersten Hautschichten), Nadeln (zum Perforieren der obersten Hautschichten) und/oder Widerhaken (zur Verankerung in der Haut) ausgestattet ist. In einer bevorzugten Ausführungsform ist das TTS jedoch ohne eine derartige Schicht ausgestattet.

In einer weiteren Ausführungsform kann das Transdermale Therapeutische System mehr als eine wirkstoffhaltige Schicht enthalten. Diese wirkstoffhaltigen Schichten können übereinander (wobei ein mindestens zweischichtiges Laminat gebildet wird) oder nebeneinander angeordnet sein. Im Fall eines solchen TTS mit mehr als einer wirkstoffhaltigen Schicht können die einzelnen Schichten gleich oder unterschiedlich aufgebaut sein. In derartigen "mehrschichtigen Systemen" unterscheiden sich diese Schichten jedoch vorzugsweise aufgrund ihrer Zusammensetzung oder des verwendeten Wirkstoffs.

Auch kann die wirkstoffhaltige Schicht in Form eines mit Flüssigkeit gefüllten Beutels bzw. einer mit Flüssigkeit gefüllten Kammer vorliegen, in der der Wirkstoff in gelöster, dispergierter oder suspendierter Form enthalten ist.

Schließlich kann der Wirkstoff in der wirkstoffhaltigen Schicht in flüssigen Mikroreservoiren enthalten sein, welche in der wirkstoffhaltigen Schicht dispergiert sind.

Mit dem hier beschriebenen TTS können vorzugsweise Peptide als Wirkstoff auf transdermalem Wege verabreicht werden. Die technische Lehre kann aber grundsätzlich auch für andere physiologisch wirksame Substanzen genutzt werden, insbesondere auch solche, die bisher der transdermalen Therapie noch nicht zur Verfügung stehen (hydrophile Wirkstoffe) oder eine Molekularmasse oberhalb von 500 Dalton besitzen.

Unter "Peptiden" im Sinne der vorliegenden Beschreibung werden durch Peptid-Bindungen säureamidartig verknüpfte Kondensationsprodukte von Aminosäuren verstanden. Bauen sich die Moleküle aus zwei Aminosäure-Resten auf, so spricht man auch von Dipeptiden, bei drei und mehr von Tripeptiden, Tetra-, Pentapeptiden etc. Peptide mit 2-10 Aminosäure-Resten fasst man daher im Allgemeinen als Oligopeptide, solche mit 10-100 als Polypeptide zusammen. Der Übergang von den letzteren zu den höhermolekularen Proteinen (Eiweißstoffen) ist jedoch nicht genau definiert. Peptide mit Bindungen zwischen den seitenständigen Amino-Gruppen von Diaminocarbonsäuren und seitenständigen Carboxy-Gruppen von Aminodicarbonsäuren statt der üblichen Peptid-Bindungen zwischen der α-Amino-Gruppe und der Carboxy-Gruppe nennt man Isopeptide; die von mehrfunktionellen Aminosäuren wie Glutaminsäure, Asparaginsäure, Lysin, Arginin ausgehenden zusätzlichen Bindungen sind für die Entstehung von Peptid-Netzstrukturen verantwortlich.

Zu den bevorzugten Peptiden zählen Peptidhormone. Es handelt sich dabei um physiologisch hochaktive Peptide, die Hormon- oder hormonähnliche Wirkung entfalten. Im allgemeinen handelt es sich bei den Peptidhormonen um Oligo- und Polypeptide (mit bis zu 100 Aminosäuren), zuweilen aber auch um höhermolekulare Proteine (Proteohormone). Hierzu zählen die glandulären Peptidhormone der Hypophyse (z.B.: Corticotropin, Follitropin, Lutropin, Melanotropin, Prolactin, Somatotropin, Thyrotropin, Oxytocin, Vasopressin), die Releasing-Hormone und inhibiting factors des Hypothalamus, die Peptidhormone aus Pankreas, Magen oder Darm (z.B.: Glucagon, Insulin, Somatostatin, Secretin, Gastrin, Cholecystokinin), aus Schilddrüse (z.B.: Calcitonin, Parathyrin). Einigen Oligopeptiden kommt sowohl klassische Hormon- als auch Wachstumsfaktor-, Neurotransmitter- oder Neuromodulator-Wirkung zu (Mediatoren). Als Beispiele hierfür sind die endogenen Opiate, Enkephaline und Endorphine zu nennen.

Die Peptide können in bevorzugter Weise in Form eines pharmazeutisch akzeptablen Salzes verwendet werden.

Neben natürlichen Peptiden und Peptidhormonen fallen auch naturidentische bzw. modifizierte (d.h. auf synthetischem Wege erzeugte) Peptide und Peptidhormone, konjugierte Proteine (d.h. Glycopeptide und Glycoproteine, Lipoproteinen, Metalloproteinen und andere zu den Peptiden im Sinne dieser Beschreibung.

Unter "Haut" ist die normale, intakte Haut eines Menschen oder Säugetiers zu verstehen. Diese ist schichtförmig aufgebaut und besteht - von außen nach innen gesehen - aus Epidermis (Oberhaut), Dermis (Lederhaut) und Subcutis (Unterhaut). Innerhalb dieser drei Bestandteile werden vom Fachmann ggf. weitere Schichten unterschieden.

Bei der Epidermis werden fünf Schichten unterschieden: Hornschicht (Stratum corneum), Glanzschicht (Stratum lucidum), Körnerschicht (Stratum granulosum), Stachelzellschicht (Stratum spinosum) und Basalschicht (Stratum basale).

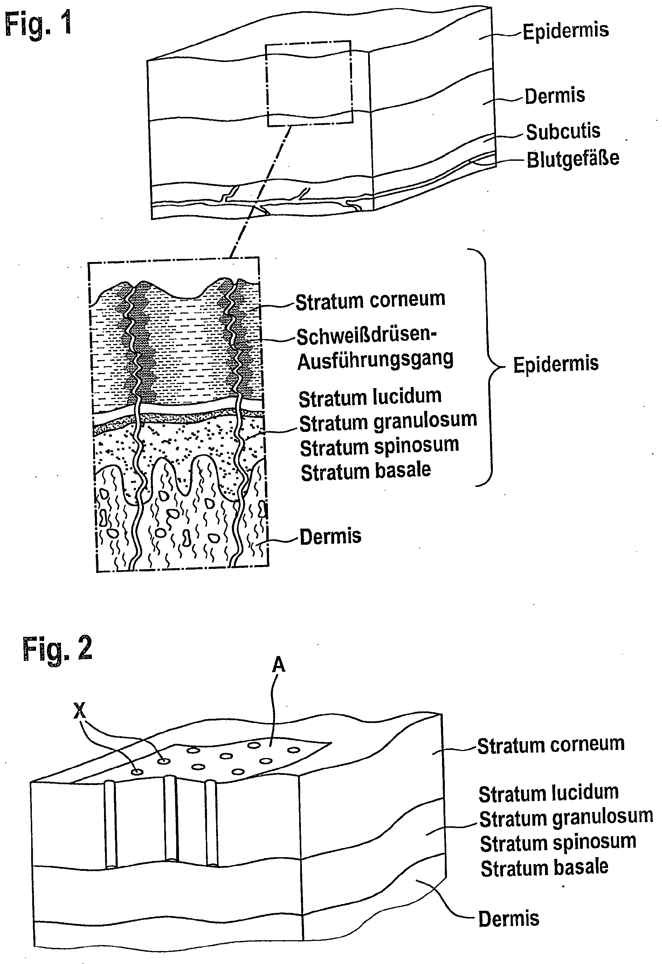

Unter "ablativ behandelter Haut" ist die normale, intakte Haut eines Menschen zu verstehen, von deren Epidermis das Stratum corneum - zumindest teilweise - zerstört oder entfernt worden ist. Dabei kann in diesem Bereich der ablativ behandelten Haut der "Flächenanteil von normaler, intakter Haut, von deren Epidermis das mindestens das Stratum corneum zerstört oder entfernt ist" (entspricht der Summe der Flächen X in Abb. 2) zur "gesamten normalen, intakten Haut, an deren Epidermis das Stratum corneum verbleibt" (entspricht der Fläche A in Abb. 2), unterhalb von 50% liegen, vorzugsweise unterhalb von 20% und besonders bevorzugt unterhalb von 10%. Die Abschnitte der Epidermis, an denen das Stratum corneum entfernt wurde, können unregelmäßig geformt sein. Vorzugsweise sind sie aber von definierter Form und Fläche. Als geeignete Formen kommen Rechtecke, Sechsecke, Achtecke, Quadrate, Kreise und Punkte in Frage. Die Abschnitte der Epidermis, die durch ablative Behandlung entfernt werden, sind so tief, dass an den betreffenden Stellen mindestens das Stratum corneum entfernt wird und so die "Mikrokanäle" unter den Flächen X (vgl. Abb. 2) entstehen. Die durch ablative Behandlung entfernten Abschnitte der Epidermis sollen vorzugsweise aber nicht tiefer reichen als bis zur Dermis. Dies kann durch eine entsprechende Anpassung der Leistung des Lasers und zeitgleich erfolgende Kontrollmessungen erreicht werden.

Als "transdermal" wird die Applikationsroute durch die Haut eines Menschen oder Säugetiers bezeichnet. Dabei ist unter Haut sowohl die normale, intakte Haut sowie die "ablativ behandelte Haut" im Sinne der vorstehenden Definition zu verstehen.

Als "Trägersubstanz" für die wirkstoffhaltige Schicht kommen Substanzen in Frage, die sich in Bezug auf das mindestens eine Peptid kompatibel verhalten. Es ist bekannt, dass sich bei Peptiden sowohl durch chemische Einflüsse, wie zum Beispiel Säuren, Salze oder organische Lösungsmittel, als auch durch physikalische Einwirkungen, wie hohe oder tiefe Temperaturen oder auch Druck, die Sekundär- und Tertiärstruktur und damit letztlich auch die Quartärstruktur verändern können (Denaturierung). Durch eine Denaturierung können sich auch die physikalischen und physiologischen Eigenschaften der Peptide ändern. Bei einer chemischen Spaltung der Peptide (Proteolyse) entstehen daraus Teilstücke, die man Peptone nennt.

Das bedeutet hinsichtlich der Anforderungen an die Kompatibilität der Trägersubstanz, dass beim Einbetten des Peptids in die Trägersubstanz keine Wechselwirkung mit dem Peptid auftreten dürfen, die zu einer solchen Veränderung der Struktur des Peptids oder zu einer auf anderen Ursachen beruhenden Verschlechterung seiner pharmakologischen Eigenschaften führen.

Die Trägersubstanz bewirkt, dass das mindestens eine Peptid in der wirkstoffhaltigen Schicht gleichmäßig verteilt ist. Vorzugsweise bewirkt die Trägersubstanz, dass die Peptidmoleküle einzeln vorliegen, d.h. in Form einer echten "Lösung".

Es hat sich herausgestellt, dass insbesondere solche Trägersubstanzen geeignet sind, die "hydrophil" sind. Unter hydrophil ("wasserliebend") versteht man die Fähigkeit, Wasser an sich zu binden bzw. in Wasser einzudringen und in einem weiteren Sinne "von Wasser gut benetzt zu werden".

Es sind also besonders solche Trägersubstanzen geeignet, die die Fähigkeit besitzen, bei Kontakt mit Wasser nicht nur zu quellen. ("Quellungsvermögen"), sondern sich in Wasser sogar lösen ("Wasserlöslichkeit"). Quellung ist der Vorgang der Änderung von Volumen und Gestalt eines Festkörpers bei Einwirkung von Wasser, wobei hier Wasser in Form einer Flüssigkeit, eines Dampfs eines Gases auftreten kann. Wenn eine unbegrenzte Quellung auftritt, geht die quellende Substanz schließlich in eine Lösung oder Suspension über, bei begrenzter Quellung bleibt sie dagegen kohärent (Gel-Bildung).

Zu den am besten geeigneten Trägersubstanzen sind also insbesondere solche zu zählen, die mindestens eine hydrophile Gruppe im Molekül aufweisen.

Konkret zählen zu den geeigneten Trägersubstanzen:
- Polyvinylpyrrolidon (PVP; z.B. Kollidon®), auch quervernetztes PVP
- Polyvinylalkohol-Polyvinylpyrrolidon-Mischpolymerisate
- Polysaccharide wie Stärke, Amylopektin, Glykogen, Inulin, Chitin, Pektine, etc.

Auch Mischungen von mindestens zwei Trägersubstanzen sind möglich.

Vorzugsweise handelt es sich bei der Trägersubstanz um eine, die bei Raumtemperatur fest ist. Flüssige Trägersubstanzen können aber auch verwendet werden.

Die Trägersubstanz kann in der wirkstoffhaltigen Schicht in Form von Fasern, Pulver oder als Film vorliegen. Bevorzugt bildet die Trägersubstanz einen Film mit einer konstanten Schichtdicke. Diese Schichtdicke kann zwischen 20 und 200 µm liegen, vorzugsweise zwischen 30 und 80 µm.

Die wirkstoffhaltige Schicht kann "Puffer" enthalten, um darin einen definierten pH-Wert aufrecht zu erhalten und die Stabilität des Wirkstoffs zu erhöhen. Puffersysteme und die damit einstellbaren pH-Werte sind dem Fachmann bekannt.

Als "Rückschicht" kommen okklusive und nicht-okklusive Schichten in Frage, wobei okklusive bevorzugt sind. Diese Schichten sind aus Folien, Geweben und/oder Gewirken aufgebaut, wobei Folien bevorzugt sind. Bei den Materialien handelt es sich um natürliche oder synthetische Polymere und Metalle. Besonders bevorzugt sind Verbundwerkstoffe aus synthetischen Polymeren und Metallen in Form von Laminaten. Die Rückschicht ist vorzugsweise flexibel und für den Wirkstoff undurchlässig.

Die "wirkstoffhaltige Schicht" enthält - wie bereits gesagt - mindestens ein Peptid und mindestens eine Trägersubstanz für das Peptid. Sie kann eine Fläche von 0,1 bis 100 cm² aufweisen, vorzugsweise von 1 bis 80 cm² und besonders bevorzugt zwischen 2 und 20 cm². Die Schichtdicke der wirkstoffhaltigen Schicht kann zwischen 20 und 200 µm liegen, vorzugsweise zwischen 30 und 80 µm.

Die "Konzentration" des mindestens einen Peptids in der wirkstoffhaltigen Schicht hängt stark von der therapeutischen Indikation, der Aktivität des jeweiligen Peptids und dessen Molekulargewicht ab. Die Konzentration kann daher in weiten Bereichen variieren und in der wirkstoffhaltigen Schicht zwischen 0,1 bis 70 Gew.-%, vorzugsweise zwischen 1 und 20 Gew.-% liegen.

Damit die wirkstoffhaltige Schicht, die ein Peptid und eine Trägersubstanz für das Peptid enthält, "haftklebend" ausgerüstet ist, kann ihr mindestens ein "Haftkleber" zugegeben werden. Die geeigneten Haftkleber sind weiter unten aufgeführt. Eine andere Möglichkeit besteht darin, durch Zugabe von Weichmachern, Tackifiern etc., die wirkstoffhaltige Schicht haftklebend auszurüsten. Insbesondere, wenn die Trägersubstanz stark hydrophil ist, ist die Verwendung von hydrophilen Klebrigmachern wie Pantothenylalkohol, Honig, niedermolekularen Kohlenhydraten (wie Saccharose, Glucose, Fructose) und deren Derivaten (wie beispielsweise Saccharoseacetatisobutyrat) und deren Kombinationen vorteilhaft.

Die wirkstoffhaltige Schicht kann in einer besonderen Ausführungsform Wasser enthalten. Vorzugsweise ist der Wassergehalt (Restfeuchtigkeitsgehalt) jedoch gering, um die mechanische Stabilität der wirkstoffhaltigen Schicht nicht zu gefährden und andere - insbesondere mikrobiologische - Risiken aufgrund der Anwesenheit von Wasser zu minimieren. Bevorzugt ist der "Wassergehalt" in der wirkstoffhaltigen Schicht unterhalb von 10%, besonders bevorzugt unterhalb von 5% und ganz besonders bevorzugt unterhalb von 3%.

Die zusätzliche "haftklebende Schicht" kann aus den dem Fachmann bekannten "Haftklebern" aufgebaut sein. Haftkleber sind in der Lage, bei Raumtemperatur ohne eine Aktivierung durch Lösungsmittel oder Wärme lediglich durch Andrücken an die Oberfläche des zu beklebenden Gegenstands eine "Benetzung" herbeizuführen, die ausreichende Haftungskräfte ergibt.

Als "Haftkleber" können "Polymere" eingesetzt werden, die aufgrund der Zusammensetzung ihrer Monomeren haftklebende Eigenschaften besitzen. Hierzu zählen Synthese- und Naturkautschuk, Butylkautschuk, Styrol-Butadien-Copolymere, Ethylen-Vinylacetat-Copolymere, Acrylnitril-Copolymere, Polychloropren, Polyisobutylen, Polyvinylether, Styrol-Butadien-Styrol-Blockpolymere, Styrol-Isopren-Styrol-Blockpolymere, Polyacrylate, Polyester, Polyurethane und Polysiloxane. Durch funktionelle Gruppen in den Monomeren dieser Polymere können die Klebereigenschaften des bei der Polymerisation erhaltenen Polymers modifiziert werden.

Eine weitere Möglichkeit, die Klebereigenschaften dieser genannten Polymere zu modifizieren bietet die Anpassung der Kleberrezeptur an die gewünschten Eigenschaften durch Zugabe von Zusatzstoffen wie Harzen, Weichmachern, Klebrigmachern, Füllstoffen und / oder Stabilisatoren.

Besonders gut geeignete Polymere mit haftklebenden Eigenschaften sind Polyacrylate, Polyisobutylene, Silikone.

Vorzugsweise werden solche Haftkleber verwendet, die sich durch ihre gute Materialverträglichkeit gegenüber den Peptiden auszeichnen und zugleich keine Hautirritationen, Allergien oder Sensibilisierung bei der Anwendung auslösen.

Als "Schutzfolie" können in dem Transdermalen Therapeutischen System die dem Fachmann bekannten Folien eingesetzt werden, wie z. B. silikonisierte Polyesterfolien.

Vor der Applikation des Transdermalen Therapeutischen Systems (TTS), welches eine wirkstoffhaltige Schicht mit mindestens einem Peptid und mindestens einer Trägersubstanz für das Peptid enthält, wird die Hornschicht (das Stratum corneum) der Haut zumindest abschnittsweise entfernt, vorzugsweise mittels der skin-ablation-Technik. In einer bevorzugten Ausführungsform weist diese ablativ behandelte Haut in diesem Areal Mikrokanäle innerhalb des Stratum corneums auf.

Durch anschließende Applikation des TTS wird die transdermale Resorption des Peptids ermöglicht. Dazu wird das TTS direkt auf die ablativ behandelte Haut platziert. Die wirkstoffhaltige Schicht, die das Peptid und eine Trägersubstanz für das Peptid enthält, kommt dabei direkt oberhalb der ablativ behandelten Haut zum liegen.

Aufgrund der zumindest punktuellen Entfernung des Stratum corneums kann das Peptid die
darunter liegenden Hautschichten erreichen und schließlich auf dem transdermalen Weg in den Blutkreislauf eintreten. Feuchtigkeit, die aus den unter dem Stratum corneum liegenden Hautschichten stammt, kann dabei den Transport des Peptids durch die zumindest punktuell entfernten Abschnitte des Stratum corneums (d.h. durch die Mikrokanäle) erleichtern.

Mit Hilfe der zusätzlichen haftklebenden Schicht kann gegebenenfalls eine zusätzliche Fixierung des TTS auf der Haut erfolgen.

In einer besonderen Ausführungsform wird während der Anwendung der "Skin-ablation-Technik" das ablativ behandelte Hautareal farblich markiert, so dass das nachfolgende Anbringen des TTS exakt und leicht ausführbar ist.

Die Applikationsdauer einer Anwendung kann von wenigen (beispielsweise 2 bis 6) Stunden bis zu einem bis mehreren (beispielsweise 3 bis 7) Tagen erfolgen. Auch sind wiederholte Anwendungen möglich. Hierzu kann das TTS auf die ablativ behandelte Haut platziert werden, auf der bereits zuvor ein TTS appliziert gewesen ist. Vorzugsweise wird das TTS - insbesondere bei einer länger andauernden therapeutischen Anwendung - stets auf eine unmittelbar zuvor ablativ behandelte Hautfläche platziert.

Mit dem Transdermalen Therapeutisches Systems (TTS) umfassend eine Rückschicht und eine wirkstoffhaltige Schicht enthaltend ein Peptid und eine hydrophile Trägersubstanz zur Verabreichung eines Peptids durch ablativ behandelte Haut an einen Patienten kann eine Person, welche unter Prostatakrebs, Endometriose oder frühzeitiger Pubertät leidet, behandelt werden, wobei es sich bei dem Peptid um Triptorelin handelt.

Mit dem Transdermalen Therapeutisches Systems (TTS) umfassend eine Rückschicht und eine wirkstoffhaltige Schicht enthaltend ein Peptid und eine hydrophile Trägersubstanz zur Verabreichung eines Peptids durch ablativ behandelte Haut an einen Patienten kann eine Person, die eine assistierende Fertilitätstherapie benötigt, behandelt werden, wobei es sich bei dem Peptid um Triptorelin handelt.

Mit dem Transdermalen Therapeutisches Systems (TTS) umfassend eine Rückschicht und eine wirkstoffhaltige Schicht enthaltend ein Peptid und eine hydrophile Trägersubstanz zur Verabreichung eines Peptids durch ablativ behandelte Haut an einen Patienten kann eine Person, die ein Antidiuretikum benötigt, die unter Enuresis nocturna leidet, die ein Antihämorrhagikum benötigt, unter Hämophilie leidet, unter urämischer Thombozytopathie leidet oder unter dem Willebrand-Jürgens-Syndrom leidet, behandelt werden, wobei es sich bei dem Peptid um Desmopressin handelt.

Mit dem Transdermalen Therapeutisches Systems (TTS) umfassend eine Rückschicht und eine wirkstoffhaltige Schicht enthaltend ein Peptid und eine hydrophile Trägersubstanz zur Verabreichung eines Peptids durch ablativ behandelte Haut an einen Patienten kann eine Person, die eine stark blutdrucksteigernde Substanz benötigt oder unter Diabetes insipidus centralis leidet, behandelt werden, wobei es sich bei dem Peptid um Vasopressin handelt.

Das Verfahren zur Herstellung eines Transdermales Therapeutisches System (TTS) zur Verabreichung von Peptiden, welches eine wirkstoffhaltige Schicht enthält, die mindestens ein Peptid und mindestens eine Trägersubstanz für das Peptid enthält, umfasst mehrere Schritte.

In ersten Schritt wird das Peptid gelöst, vorzugsweise in einen entsprechenden Puffer. Als besonders gut geeignete Lösungsmittel kommen isotonische Kochsalzlösung und wäßrige Puffer-Lösungen mit einem entsprechendem pH-Wert in Frage.

Dann wird die Trägersubstanz ebenfalls gelöst, wobei Lösungsmittel wie Ethanol, Wasser, niedrigsiedene Lösungsmittel in Frage kommen. Die beiden Lösungen werden gemischt.

Weitere Hilfssubstanzen können zugegeben werden, wie z.B. Stabilisatoren (beispielsweise Mannitol), Haftkleber, Weichmacher, Tackifier etc. - entweder in eine der beiden Lösungen (d.h. Peptidlösung oder Trägersubstanzlösung) oder in die Mischung der beiden Lösungen.

Die erhaltene Masse kann auf einer Unterlage ausgestrichen werden mit einer Schichtdicke von 10 bis 500 µm.

In einen weiteren Arbeitsschritt wird die so erhaltene schichtförmige Masse getrocknet um die Lösemittel zu entfernen, vorzugsweise bis zu einem gewünschten restlichen Wassergehalt unterhalb von 20%, bevorzugt unterhalb von 10%.

Aus der so erhaltenen wirkstoffhaltigen Schicht können einzelne Abschnitte ausgestanzt werden, die durch Laminieren mit der Rückschicht und der Schutzschicht verbunden werden.

Alternativ können auch die weiteren Komponenten des TTS, insbesondere die Rückschicht und die Schutzfolie, durch Laminieren mit der zuvor hergestellten wirkstoffhaltigen Schicht verbunden werden.

In einer bevorzugten Ausführungsform wird die wirkstoffhaltige Schicht - ggf. auch in Form einzelner Abschnitte - auf eine zusätzliche Haftkleberschicht gelegt und anschließend mit den weitern Komponenten des TTS - insbesondere der Rückschicht und der Schutzfolie - durch Laminieren verbunden.

Die nachfolgenden Beispiele dienen der Veranschaulichung der Erfindung, ohne sie einzuschränken.

### Beispiel 1:

Triptorelinacetat wird in einer wässrigen Acetatpufferlösung (pH 5,0) gelöst. Zu dieser Lösung wird soviel Mannitol gegeben, bis dieses in einer Konzentration von 3% Mannitol vorliegt. Nach Zugabe einer ethanolischen Polyvinylpyrrolidon-Lösung (Kollidon 90 F) und Glycerin erhält man eine Masse, aus der durch Beschichten und Trocknung der Lösungsmittel (Wasser, Ethanol) ein gleichmäßig dicker Film hergestellt wird.

Die nachfolgende Tabelle gibt die Zusammensetzung der resultierenden wirkstoffhaltigen Schicht in getrocknetem Zustand wieder.

| wirkstoffhaltige Schicht | Menge [in g] | Menge [in %] |
|---|---|---|
| Triptorelin Acetat | 3,39 | 16,95 |
| Polyvinylpyrrolidon (in Ethanol gelöst) | 13,47 | 67,35 |
| Glyzerin | 2,00 | 10,00 |
| Natriumacetat (Trihydrat) | 0,48 | 2,40 |
| Essigsäure | 0,12 | 0,60 |
| Mannitol | 0,54 | 2,70 |
| Aqua purificata | --- | --- |
| Summe: | 20,00 | 100 |

Die Schichtdicke beträgt etwa 40 µm.

Aus dem getrockneten Film werden quadratische Flächenabschnitte von 5 cm² ausgeschnitten, die einen Gehalt von 3 mg Triptorelin aufweisen. Dies entspricht einer Beladung von 0,6 mg/cm².

Diese Flächenabschnitte aus der wirkstoffhaltigen Schicht werden mittig auf ein 10 cm² großes, quadratisches Stück einer Rückschicht aufgelegt, die auf ihrer Unterseite mit einer 60 µm dicken zusätzlichen Haftkleberschicht ausgerüstet ist. Bei dem Material der Haftkleberschicht handelt es sich um ein Gemisch aus 85 Gew.-% eines hochmolekularen Polyisobutylens (Oppanol B 100) und 15 Gew.-% eines mittelmolekularen Polyisobutylens (Oppanol B 10).

Zur Abdeckung der wirkstoffhaltigen Schicht und der überstehenden Ränder der Haftkleberschicht wird eine silikonisierte Polyesterfolie verwendet.

### Beispiel 2:

Muster gemäß Beispiel 1 werden hergestellt mit dem Unterschied, dass die Beladung mit Triptorelin-Acetat 0,1 mg/cm², 0,2 mg/cm² bzw. 0,3 mg/cm² entspricht.

### Beispiel 3:

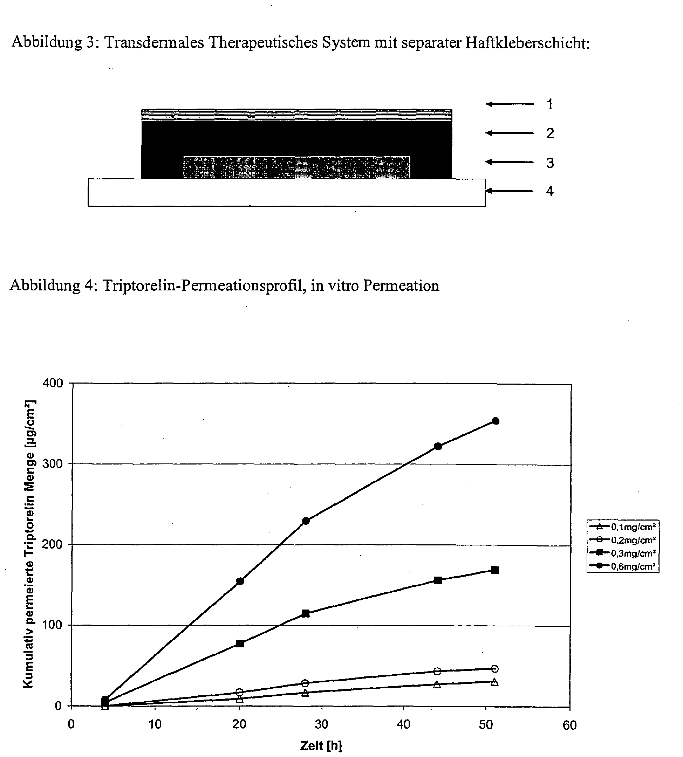

Muster der in Beispiel 1 und 2 hergestellten Transdermalen Therapeutischen Systeme mit Triptorelinacetat als Wirkstoff werden in einer Franz'schen Diffusionszelle hinsichtlich ihres Permeationsverhaltens untersucht. Die Ergebnisse dieser Untersuchungen sind in Abb. 4 dargestellt, wobei als Modellmembran eine mittels Laser ablativ vorbehandelte Kuheuterhaut verwendet wurde.

### Beschreibung der Abbildungen

Abbildung 1 zeigt den schematischen Aufbau intakter, normaler Haut mit einem vergößertern Ausschnitt der äußersten Schicht. Darin bedeuten:
   - E: = Epidermis
   - D: = Dermis
   - S: = Subcutis
   - B: = Blutgefäß
   - s.c.: = Stratum corneum
   - s.l.: = Stratum lucidum
   - s.gr.: = Stratum granulosum
   - s.sp.: = Stratum spinosum
   - s.b.: = Stratum basale
Abbildung 2 zeigt den schematischen Aufbau ablativ behandelter Haut. Hier bedeuten A das ablativ behandelte Hautareal und X die Flächen, an denen das Stratum corneum entfernt wurde.
Abbildung 3 zeigt den schematischen Aufbau eines Transdermalen Therapeutischen Systems gemäß Beispiel 1. Es bedeuten: 1 = Rückschicht, 2 = Haftkleberschicht, 3 = wirkstoffhaltige Schicht, 4 = Schutzfolie.
Abbildung 4 zeigt den Einfluss der Triptorelinmenge auf die in vitro-Permeation nach den Beispielen 1 und 2. Als Permeationsbarriere für die Untersuchungen diente eine ablativ behandelte 1200µm dicke dermatomisierte Kuheuterhaut.

## Patentansprüche

1. Transdermales Therapeutisches System (TTS) zur Verabreichung eines Peptids auf ablativ behandelte Haut an einen Patienten, umfassend eine Rückschicht, eine Schutzfolie und mindestens eine wirkstoffhaltige Schicht, die mindestens ein Peptid und eine hydrophile, wasserlösliche Trägersubstanz enthält, die ausgewählt ist aus der Gruppe bestehend aus Polyvinylpyrrolidon, insbesondere ein Polyvinylpyrrolidon mit einem Molekulargewicht zwischen 790.000 bis 1.500.000 Da, quervernetztes Polyvinylpyrrolidon, Polyvinylalkohol-Polyvinylpyrrolidon-Mischpolymerisaten, Polysacchariden und Mischungen davon, wobei die wirkstoffhaltige Schicht einen Wassergehalt von unterhalb 20 % aufweist.

2. TTS nach Anspruch 1, **dadurch gekennzeichnet, dass** das Peptid ein Oligopeptid, ein Polypeptid, ein Protein, ein Isopeptid, ein Peptidhormon oder eine Kombination davon ist.

3. TTS nach Anspruch 1 oder 2, **dadurch gekennzeichnet, dass** es sich bei dem Peptid um ein glanduläres Peptidhormon der Hypophyse, ein releasing-hormone des Hypothalamus, ein inhibiting factor des Hypothalamus, ein Peptidhormon aus dem Pankreas, ein Peptidhormon aus dem Magen oder ein Peptidhormon aus dem Darm oder um ein pharmazeutisch akzeptables Salz dieses Peptids handelt.

4. TTS nach einem oder mehreren der Ansprüche 1 bis 3, **dadurch gekennzeichnet, dass** es sich bei dem Peptid um Triptorelin, Desmopressin oder Vasopressin oder um ein pharmazeutisch akzeptables Salz davon handelt.

5. TTS nach einem oder mehreren der Ansprüche 1 bis 4, **dadurch gekennzeichnet, dass** das Peptid in einer Konzentration von 0,1 bis 70 Gew.-%, vorzugsweise zwischen 1 und 20 Gew.-% in der wirkstoffhaltigen Schicht enthalten ist.

6. TTS nach einem oder mehreren der Ansprüche 1 bis 5, **dadurch gekennzeichnet, dass** die wirkstoffhaltige Schicht einen Wassergehalt unterhalb von 10 % und vorzugsweise unterhalb von 5 % aufweist.

7. TTS nach einem oder mehreren der Ansprüche 1 bis 6, **dadurch gekennzeichnet, dass** die wirkstoffhaltige Schicht haftklebend ausgerüstet ist und / oder zusätzlich mindestens eine Haftkleberschicht enthält, die vorzugsweise zwischen der wirkstoffhaltigen Schicht und der Rückschicht angeordnet ist und die die wirkstoffhaltige Schicht an zumindest einem Abschnitt entlang ihres/ihrer seitlichen Rands/Ränder überragt.

8. TTS nach einem oder mehreren der Ansprüche 1 bis 7, **dadurch gekennzeichnet, dass** es eine die Geschwindigkeit des Austritts des Wirkstoffs aus der wirkstoffhaltigen Schicht kontrollierende Membran besitzt.

9. TTS nach einem oder mehreren der Ansprüche 1 bis 8, **dadurch gekennzeichnet, dass** die wirkstoffhaltige Schicht eine Fläche von 0,1 bis 100 cm², vorzugsweise von 1 bis 80 cm² und besonders bevorzugt von 2 bis 20 cm², sowie eine Dicke zwischen 20 und 200 µm, vorzugsweise zwischen 30 und 80 µm aufweist.

10. Verfahren zur Herstellung eines TTS umfassend eine Rückschicht, eine wirkstoffhaltige Schicht, die mindestens ein Peptid und eine hydrophile Trägersubstanz enthält, die ausgewählt ist aus der Gruppe bestehend aus Polyvinylpyrrolidon, insbesondere ein Polyvinylpyrrolidon mit einem Molekulargewicht zwischen 790.000 bis 1.500.000 Da, quervernetztes Polyvinylpyrrolidon, Polyvinylalkohol-Polyvinylpyrrolidon-Mischpolymerisaten, Polysacchariden und Mischungen davon, und eine Schutzfolie,
durch die Schritte:
a. Mischen des Peptids mit einer Lösung der hydrophilen Trägersubstanz,
b. Ausstreichen der so erhaltenen Masse in einer konstanten Schichtdicke auf eine Unterlage,
c. Trocknen der ausgestrichen Masse bis zu einem vorbestimmten Wassergehalt unterhalb von 20 %, vorzugsweise unterhalb von 10 % unter Bildung der wirkstoffhaltigen Schicht, und
d. Herstellen einzelner Abschnitte der wirkstoffhaltigen Schicht und Verbinden mit der Rückschicht.

11. Verfahren nach Anspruch 10, **dadurch gekennzeichnet, dass** die einzelnen Abschnitte der wirkstoffhaltigen Schicht mittels einer zusätzlichen Haftkleberschicht auf der Rückschicht verbunden werden.

## Claims

1. Transdermal therapeutic system (TTS) for administering a peptide onto ablatively treated skin to a patient, comprising a backing layer, a protective sheet and at least one active ingredient layer, which comprises at least one peptide and a hydrophilic, water-soluble carrier substance which is selected from the group consisting of polyvinylpyrrolidone, more particularly a polyvinylpyrrolidone having a molecular weight between 790 000 and 1 500 000 Da, crosslinked polyvinylpyrrolidone, polyvinyl alcohol-polyvinylpyrrolidone copolymers, polysaccharides, and mixtures thereof, wherein the active ingredient layer has a water content below 20 %.

2. TTS according to Claim 1, **characterized in that** the peptide is an oligopeptide, a polypeptide, a protein, an isopeptide, a peptide hormone or a combination thereof.

3. TTS according to Claim 1 or 2, **characterized in that** the peptide is a glandular peptide hormone of the hypophysis, a releasing hormone of the hypothalamus, an inhibiting factor of the hypothalamus, a peptide hormone from the pancreas, a peptide hormone from the stomach or a peptide hormone from the gut or a pharmaceutically acceptable salt of this peptide.

4. TTS according to one or more of Claims 1 to 3, **characterized in that** the peptide is triptorelin, desmopressin or vasopressin or a pharmaceutically acceptable salt thereof.

5. TTS according to one or more of Claims 1 to 4, **characterized in that** the peptide present in a concentration of 0.1 to 70 % by weight, preferably between 1 and 20 % by weight in the active ingredient layer.

6. TTS according to one or more of Claims 1 to 5, **characterized in that** the active ingredient layer has a water content below 10 % and preferably below 5 %.

7. TTS according to one or more of Claims 1 to 6, **characterized in that** the active ingredient layer is made pressure-sensitively adhesive and/or additionally comprises at least one layer of pressure-sensitive adhesive which is preferably disposed between the active ingredient layer and the backing layer and which protrudes beyond the active ingredient layer in at least one section along the side margin/margins thereof.

8. TTS according to one or more of Claims 1 to 7, **characterized in that** it possesses a membrane which controls the rate of emergence of the active ingredient from the active ingredient layer.

9. TTS according to one or more of Claims 1 to 8, **characterized in that** the active ingredient layer has an area of 0.1 to 100 cm², preferably of 1 to 80 cm², and more preferably from 2 to 20 cm², and a thickness of between 20 and 200 µm, preferably between 30 and 80 µm.

10. Method for producing a TTS comprising a backing layer; an active ingredient layer which comprises at least one peptide and a hydrophilic carrier substance which is selected from the group consisting of polyvinylpyrrolidone, more particularly a polyvinylpyrrolidone having a molecular weight between 790 000 and 1 500 000 Da, crosslinked polyvinylpyrrolidone, polyvinyl alcohol-polyvinylpyrrolidone copolymers, polysaccharides, and mixtures thereof, wherein the active ingredient layer has a water content below 20 %; and a protective sheet,
by the steps of:
a. mixing the peptide with a solution of the hydrophilic carrier substance,
b. spreading the composition thus obtained in a constant thickness on an underlayer,
c. drying the spread composition to a predetermined water content of below 20 %, preferably below 10 %, to form the active ingredient layer, and
d. producing individual sections of the active ingredient layer and joining them to the backing layer.

11. Method according to Claim 10, **characterized in that** the individual sections of the active ingredient layer are joined to the backing layer by means of an additional layer of pressure-sensitive adhesive.

## Revendications

1. Système thérapeutique transdermique (TTS) pour l'administration à un patient d'un peptide sur une peau ayant subi un traitement ablatif, comprenant une couche arrière, un film de protection et au moins une couche contenant le principe actif, qui contient au moins un peptide et un véhicule hydrophile, soluble dans l'eau, qui est sélectionné dans le groupe constitué par la polyvinylpyrrolidone, en particulier une polyvinylpyrrolidone d'un poids moléculaire entre 790 000 à 1 500 000 Da, une polyvinylpyrrolidone réticulée, des copolymères alcool polyvinylique-polyvinylpyrrolidone, des polysaccharides et des mélanges de ceux-ci, dans lequel la couche contenant le principe actif présente une teneur en eau inférieure à 20 %.

2. TTS selon la revendication 1, **caractérisé en ce que** le peptide est un oligopeptide, un polypeptide, une protéine, un isopeptide, une hormone peptidique ou une combinaison de ceux-ci.

3. TTS selon la revendication 1 ou 2, **caractérisé en ce que** le peptide est une hormone peptidique glandulaire de l'hypophyse, une libérine de l'hypothalamus, un facteur d'inhibition de l'hypothalamus, une hormone peptidique du pancréas, une hormone peptidique de l'estomac ou une hormone peptidique de l'intestin ou un sel pharmaceutiquement acceptable de ce peptide.

4. TTS selon une ou plusieurs des revendications 1 à 3, **caractérisé en ce que** le peptide est de la triptoréline, desmopressine ou vasopressine ou un sel pharmaceutiquement acceptable de celles-ci.

5. TTS selon une ou plusieurs des revendications 1 à 4, **caractérisé en ce que** le peptide est contenu dans une concentration de 0,1 à 70 % en poids, de préférence entre 1 et 20 % en poids dans la couche contenant le principe actif.

6. TTS selon une ou plusieurs des revendications 1 à 5, **caractérisé en ce que** la couche contenant le principe actif présente une teneur en eau inférieure à 10 % et de préférence inférieure à 5 %.

7. TTS selon une ou plusieurs des revendications 1 à 6, **caractérisé en ce que** la couche contenant le principe actif est adhésive et/ou contient en plus au moins une couche d'adhésif, qui est agencée de préférence entre la couche contenant le principe actif et la couche arrière et qui dépasse la couche contenant le principe actif au niveau d'au moins une section le long de son/ses bord/s latéral/latéraux.

8. TTS selon une ou plusieurs des revendications 1 à 7, **caractérisé en ce qu'**il dispose d'une membrane contrôlant la vitesse de sortie du principe actif de la couche contenant le principe actif.

9. TTS selon une ou plusieurs des revendications 1 à 8, **caractérisé en ce que** la couche contenant le principe actif présente une surface de 0,1 à 100 cm², de préférence de 1 à 80 cm² et de manière particulièrement préférée de 2 à 20 cm², ainsi qu'une épaisseur entre 20 et 200 µm, de préférence entre 30 et 80 µm.

10. Procédé de fabrication d'un TTS comprenant une couche arrière, une couche contenant le principe actif, qui contient au moins un peptide et un véhicule hydrophile, qui est sélectionné dans le groupe constitué par la polyvinylpyrrolidone, en particulier une polyvinylpyrrolidone d'un poids moléculaire entre 790 000 à 1 500 000 Da, une polyvinylpyrrolidone réticulée, des copolymères alcool polyvinylique-polyvinylpyrrolidone, des polysaccharides et des mélanges de ceux-ci, et un film de protection,
par les étapes de :
a. mélange du peptide avec une solution du véhicule hydrophile,
b. étalement de la masse ainsi obtenue avec une épaisseur de couche constante sur un support,
c. séchage de la masse étalée jusqu'à une teneur en eau prédéterminée inférieure à 20%, de préférence inférieure à 10 % en formant la couche contenant le principe actif, et
d. fabrication de différentes sections de la couche contenant le principe actif et liaison à la couche arrière.

11. Procédé selon la revendication 10, **caractérisé en ce que** les différentes sections de la couche contenant le principe actif sont reliées au moyen d'une couche d'adhésif supplémentaire sur la couche arrière.
